# EUROPEAN PATENT SPECIFICATION

(11) **EP 1 871 151 B1**
(45) Date of publication and mention of the grant of the patent: **14.01.2015**
(21) Application number: 06291015.3
(22) Date of filing: 21.06.2006
(51) Int. Cl.: H05H 15/00

(54) **Method and device for creating stable and tuneable quasi monoenergetic electron beam**
Verfahren und Vorrichtung zur Erzeugung eines beständigen und abstimmbaren Strahles von nahezu mono-energetischen Elektronen
Procédé et dispositif de génération d'un faisceau d'électrons quasi mono-énergétiques stable et réglable

(43) Date of publication of application: 26.12.2007
(73) Proprietor: Ecole Polytechnique, 91120 Palaiseau (FR); Ecole Nationale Supérieure de Techniques Avancées, 75015 Paris (FR); Centre National de la Recherche Scientifique, 75016 Paris (FR)
(72) Inventor: Faure, Jérome, 94230 Cachan (FR); Malka, Victor, 75005 Paris (FR); Rechatin, Clément, 92290 Chatenay Malabry (FR); Norlin, Andreas, 26270 Strovelstorp (SE)
(74) Representative: Sartorius, Jérome

(56) References cited:
- EP-A- 1 617 713
- JP-A- 5 082 300
- US-A- 4 876 725
- US-A- 5 254 832
- US-A- 5 568 496
- US-A- 5 789 876
- US-B1- 6 358 243
- ESAREY E ET AL: "Femtosecond electron and x-ray generation by laser and plasma-based sources" PROCEEDINGS OF THE 2ND ICFA ADVANCED ACCELERATOR WORKSHOP. PHYSICS OF HIGH BRIGHTNESS BEAMS WORLD SCIENTIFIC SINGAPORE, SINGAPORE, 2000, pages 146-163, XP002406598 ISBN: 981-02-4422-3

## Description

The invention relates generally to a device and a method for high-energy electrons using a laser system producing laser pulses with pulses length shorter than 1000 fs (femtoseconds) and capable to be focused to peak intensities greater than 10^17 W/cm^2 (watts per centimeter squared) and a target device with an interaction zone is arranged to be exposed to laser pulses.

Alternatively to conventional accelerators, a method and a device for generating a collimated beam of high-energy particles, such as electrons, using laser-plasma interaction is disclosed for instance in document US 2002/0172317A1 by Maksimchuk et al., in document US 2003/01383774A1 by Tajima, and in document Medical Physics, vol. 31(7), pp. 2053 - 2067 (2004) by Kainz et al.. In comparison to conventional accelerators the spectral energy distribution of the electrons is very broad and therefore not well appropriate for novel applications.

More advanced accelerators are described in document "A laser-plasma accelerator producing monoenergetic beams" in Nature, vol. 431, pp. 541 - 544 (2004), and in document EP 1 617 713 A1, both documents are hereby entirely incorporated by reference into this specification. By focusing an ultra-intense and ultra-short laser pulse onto a material target under certain conditions, an underdense plasma can be produced. It is possible to generate a very strong electrical field, more than a few hundred GV/m (gigavolt per meter), capable to accelerate particles, in particular electrons, from the plasma to high energies and into a collimated and pulsed beam on a very short length scale in comparison to conventional particle accelerators, such as cyclotrons or the like. Basically, in response to the impinging powerful laser pulse, electrons are accelerated to relativistic energies and ejected from the plasma.

From the theoretical and simulations papers by Esarey et al., Phys. Rev. Lett., vol. 79, 2682 (1997), by Schroeder et al. Phys. Rev. E 59, 6037 (1999), and by G. Fubbiani et al., Phys. Rev. E., vol. 70, 016402-1 (2004) it is predicted that a very strong electrical field is generated, more than a few GV/m (gigavolt per meter), by focusing a first laser pulse into a target, and interacting with a second laser pulse focused into the created plasma for injecting and accelerating of electrons.

A laser-plasma-based source of relativistic electrons based on colliding laser pulses is described in document US 5,789,876. A first ultra-short laser pulse creates a wakefield plasma wave in a target, a second ultra-short laser pulse which partially overlaps the first laser pulse in the interaction zone of the target acts on electrons so that they become trapped in the plasma wave and accelerated to relativistic velocities. The laser pulses are both generated from the same laser system. Laser pulse energies are typically of the order of 1 to 5 J (Joules) with pulse width of 100 fs (femtoseconds) at a wavelength of about 1 µm (micrometer).

It is an object of the present invention to practically exploit the mentioned theoretical predictions in providing a device and an method for producing high-energy electrons with a peaked energy distribution for accelerators physics, material science, ultra fast phenomena and for cancer treatment, in particular in intensity modulated radiation therapy (IMRT).

The technical problem to solve is to conceive a device and a method for creating high-energy electrons capable to achieve a fixed and precise timing between the laser pulses to collide at a convenient location.

This problem is solved by a device defined in claim 1 and/or by a method defined in claim 13. Further improvements and advantageous embodiments and refinements are defined by the limitations set out in the dependent claims.

According to the invention a device for creating high-energy electrons (in particular a bunch, a pulse or a beam) is provided which comprises a laser system producing laser pulses, in particular a first laser pulse and a second laser pulse, with pulse length shorter than 1000 fs (femtoseconds), preferred shorter than 100 fs or even 70 fs, especially shorter than or equal to 50 or even 30 fs, and capable to be focused to peak intensities greater than 10^17 W/cm^2, preferred greater than 10^18 or even 10^19 W/cm^2 (watts per centimeter squared). At least one target device (preferably one target device) with an interaction zone is arranged to be exposed to laser pulses. The device comprises also delivery optics having components being capable to guide or to convey a first laser pulse and a second laser pulse which both are produced by the laser system to the at least one target device in such a way that the first laser pulse and the second laser pulse at least partially overlap (or collide or beat or interfere), preferably substantially or totally overlap, in the interaction zone of the target device thereby releasing high-energy electrons from the interaction zone. The location of the target device is tuneable (or variable or settable) for changing the location of the interaction zone where the first laser pulse and the second laser pulse at least partially overlap. Two laser pulses overlap (or collide or beat or interfere) at least partially when there is at least one moment or time interval and at least one spatial point or volume in which the two laser pulses meet both with a higher intensity than their respective maximal intensity over e^2. Alternatively to this description of overlapping, two laser pulses overlap at a certain spatial point or position during the time when for both of the laser pulses the time interval extending three times the time the intensity has fallen to half of the peak intensity before and after the passage of the peak intensity occurs.

According to the invention a method for creating high-energy electrons is provided in which laser pulses, in particular a first laser pulse and a second laser pulse, with a pulse length shorter than 1000 fs (femtoseconds), preferred shorter than 100 fs or even 70 fs, especially shorter than or equal to 50 or even 30 fs, and capable to be focused to peak intensities greater than 10^17 W/cm^2, preferred greater than 10^18 or even 10^19 W/cm^2 (watts per centimeter squared) are produced. At least one target device (preferably one target device) with an interaction zone arranged to be exposed to laser pulses is provided. A first laser pulse and a second laser pulse which both are produced by the laser system are guided to the at least one target device in such a way that the first laser pulse and the second laser pulse at least partially overlap (or collide or beat or interfere), preferably substantially or totally overlap, in an interaction zone of the target device thereby releasing high-energy electrons from the interaction zone.

The general physical principle forming the basis of the present invention is to use the beating of the laser pulses and/or the beating of the consequent plasma waves driven by the laser pulses to control the injection of electrons in the plasma wave. In one particular mode, there are only exactly two pulses colliding at one moment (an interaction event) in the interaction zone. In another particular mode, there are further laser pulses, preferred a third laser pulse, which are also created by the laser system and further components of the delivery optics are arranged to guide these laser pulses, in particular the third laser pulse to the interaction zone. In this another particular mode the third laser pulse can arrive before the first and the second laser pulse without overlapping with these or substantially simultaneously with the first and the second laser pulse, in consequence, at least partially overlapping. In other words, the device according to the invention can comprise further components of the delivery optics capable to guide at least a third laser pulse which is also produced by the laser system to the at least one target device in such a way that the third laser pulse either arrives before the first laser pulse and the second laser pulse or at least partially overlaps in the interaction zone of the target device with the first laser pulse and/or the second laser pulse. At least one of the laser pulses can create a plasma in the interaction zone. The interaction zone can also be designated by the term electron source. The delivery optics can comprise different light paths for the first and the second laser pulse. It can comprise light guiding elements like flat mirrors, curved mirrors or the like.

Using this device or this method according to the invention it is advantageously possible to produce at least one, preferred a plurality of high-energy electron pulses or a high-energy electron beam. The electrons have a peaked energy distribution, they can be quasi-monoenergetic, e.g. can have a narrow or small energy variance. The electrons can have an energy greater than 1 MeV. In particular the peak energy of the accelerated or released electrons can vary from 20 to 300 MeV. In particular, the electron pulse is collimated featuring a small emittance or divergence, in particular a divergence angle smaller than 10 mrad, preferred 5 mrad, for the full width half maximum of the pulse and an energy spread smaller than 5 %, preferred 1%. The electrons beam is then quasi-monoenergetic, in particular monoenergetic, with a narrow energy distribution. In particular the electron pulse has a duration shorter than the laser pulses. The two laser pulses have preferably the same polarisation, in particular circular or linear polarisation.

The device and the method according to the invention need only one laser system capable to emit ultra-short ultra-intense laser pulses which is commercially very advantageous due to a minimised amount of capital to invest and minimised operating and maintenance costs. Furthermore, as a technical advantage in practice, from one laser system it is possible to create a first laser pulse and a second laser pulse with a precise or exact delay between each other which is maintained during the run of the laser pulses through different light paths. In consequence, due to the easy and precise handling of the guiding or delivery optics, the laser pulses can be delivered or conveyed substantially, in particular exactly simultaneously to the intersection of the light paths of the two laser pulses where they overlap and the interaction zone of the target device is positioned.

The delivery optics can be tuneable (or variable or settable) for changing the temporal and/or spatial overlap of the first laser pulse and the second laser pulse in the interaction zone. The delivery optics can comprise a delay line for adjusting the arrival time of one of the first and the second laser pulse with respect to the other of the first and the second laser pulse in the interaction zone. Alternatively, a delay line can be arranged in the laser system after or before compression of the laser pulses. In this advantageous way the energy of the released electrons can be tuned in a certain range.

In a preferred embodiment the first laser pulse and the second laser pulse are substantially counter-propagating through the interaction zone. In other words the propagation direction of the first laser pulse and the propagation direction of the second laser pulse differ by essentially 180 degrees, e.g. from 160 to 200 degree, in particular 170 to 210 degree, when the laser pulses traverse the interaction zone.

Furthermore in a preferred embodiment the device according to the invention can comprise one blocking device or a plurality of blocking devices (an optical diode) for avoiding feedback of laser energy and/or entry of laser energy counter to a light path of a laser pulse through the delivery optics into the laser system or into parts of the laser system upstream from a compressor. Such a blocking device is particularly useful in a counter-propagating geometry. In concrete embodiments the blocking device can for instance be a Faraday rotator or a Pockels cell. The one or the plurality of blocking devices can be arranged either in the laser light paths in or before the delivery optics or inside the laser system, in particular upstream from a compressor optics.

In a preferred embodiment the laser system of the source according to the invention is a chirped pulse amplification (CPA) facility, in particular a double-CPA laser system, of a self mode-locked Ti:Sapphire laser with output energy greater than 1.6 J (0.6 J on target), output power greater than 20 TW, especially greater than 100 TW, and can have a repetition rate greater than 5 Hz, preferably especially equal to or greater than 10 Hz, the laser being capable of emitting laser pulses shorter than 40 fs, preferably shorter than or equal to 30 fs. In addition the laser system can comprise a device, in particular an adaptive optics, for shaping the temporal intensity profile or the spatial profile as disclosed in document EP 1 617 713 A1. This adaptive optics device can be a part of the laser system itself or might be acting on laser pulses leaving the laser system before the interaction with the target takes place.

In a particular convenient embodiment the device comprises a control unit capable to change the temporal and/or spatial overlap of the first and the second laser pulse in the interaction zone by changing the length and/or the run of at least one of the light paths of the first and the second laser pulses through the delivery optics. The control unit can be the laser control unit or a part of the laser control unit. The electron injection location is then controlled by the location in the target device where the two laser pulses overlap. In particular, when the two laser pulses are not exactly counter-propagating the overlap of the two laser pulses is controlled both by changing temporal and the spatial position of the laser pulses.

In addition or alternatively to the other mentioned features, the first laser pulse and the second laser pulse can be created out of one amplified laser pulse of the laser system by splitting the amplified laser pulse into two pulses before pulse compression (before the compressor optics). In other words, the laser system can comprise a beam splitter arranged in the light path after the amplification stages (when the pulse is still stretched) and two compressor optics, one for each output light path. The compressor optics can comprise a pair of gratings. The beam splitter can be dielectric. The laser system can comprise in particular a tuneable beam splitter having a tuneable splitting ratio in order to provide two laser pulses with different duration and/or different energies. If there are further laser pulses, in particular a third laser pulse, from the laser system to be used in the interaction zone, this third or these further pulses can be created system by splitting the amplified laser pulse into three or more pulses before compression (before the compressor optics) of each of the pulses.

In a particular suitable embodiment for the peak intensity of the first is preferentially more than 3 times, or greater than the peak intensity of the second laser pulse and/or the normalised vector potential of the first is preferentially more than 1.5 times, or greater than the normalised vector potential of the second laser pulse. Furthermore it is preferred that the first laser pulse and the second laser pulse have the same duration.

The target device in the interaction zone can comprise a gas target and/or a plasma target, in particular comprising hydrogen or helium, with a density for which no self injection by one laser pulse is dominant. Preferably, the density is adjustable. In different embodiments of the invention the at least one electron source in the target device can be (i) a fluid in particular a gas jet, a gas cell, a capillary filled target or a capillary gas filled tube or (ii) a plasma, a plasma channel, a plasma discharge or a plasma capillary discharge. In an advantageous embodiment the gas jet is a supersonic jet, preferably a supersonic Helium gas jet. The gas jet, in particular the supersonic Helium gas jet, can have more than 1 mm length and/or can provide an initial plasma electron density of the order of 10^17 electrons / cm^3, in particular of 1-5 x 10^18 electrons / cm^3. The parameters of the material in the interaction zone can be adjustable and/or controlled. Preferably, said adjustment or control is performed by the control unit, in particular the laser control unit.

The device and the method according to this specification provide high-energy electrons which can broadly and advantageously be used in medical applications, radiological applications, radiobiological applications, radiochemical applications, or applications in physical engineering, or in material engineering, e. g. non-destructive material or mechanical inspection.

Further improvements, refinements and advantageous embodiments, features and characteristics are described below and explained in more detail by referring to the attached drawings. It should be understood that the detailed description and specific examples given, while indicating the preferred embodiment, are intended for purpose of illustration and are not intended to unduly limit the scope of the present invention, which is determined by the appended claims.

The various features, advantages and possible uses of the present invention will become more apparent in the following description and the attributed drawings, wherein:
Figure 1 is showing a schematic representation of the topology of a preferred embodiment of the device for creating high-energy electron pulses using two colliding laser pulses,
Figure 2 is showing a scheme of the preferred embodiment of the chirped pulse amplification (CPA) laser facility used in the device according to the invention, and
Figure 3 is showing measured results on the tuneability of the high-energy electrons achieved using a setup having the topology shown in Figure 1.

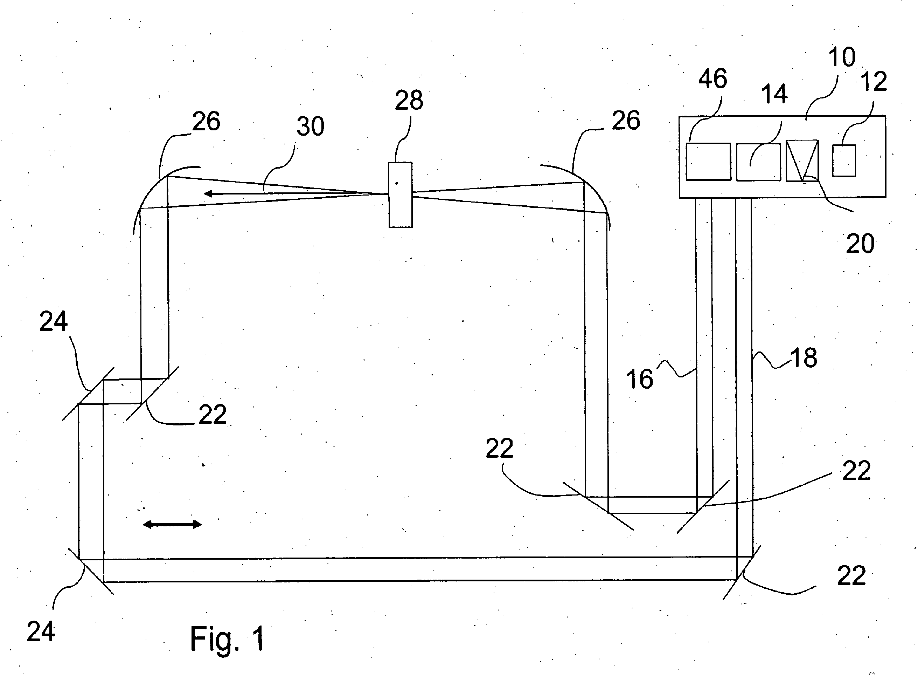

In Figure 1 a schematic representation of the topology of a preferred embodiment of the device for creating the high-energy electron collimated pulse is shown. A laser system 10 is capable of emitting a train of sub-picosecond ultra-intense laser pulses shorter than 200 fs, in this embodiment 30 fs, which can be focused to peak intensities greater than 10^17 W/cm^2, in this embodiment to about 10^18 W/cm^2. An amplified laser pulse 12 can be separated into two laser pulses by using a beam splitter 14. The output of the laser system 10 consists after compression of each of the splitted pulses of a first laser pulse 16 and a second laser pulse 18 which have an advantageously steep rising edge and a flat front phase corrected with adaptive optics. A laser pulse 16,18 contains about 1 J energy at 820 nm central wavelength. The laser system 10 comprises also a blocking device 20, an optical diode, so that only light from the amplification upstream can pass downstream, while light travelling upstream counter to the laser direction is blocked in order to avoid parts of the laser system (10) upstream from a compressor 46. Alternatively to the situation shown in Figure 1, in each of the light paths of the first and the second laser pulse 16,18 a blocking device 20 can be arranged so that only light from the laser system 10 towards the target device 28 can pass while light in the counter direction is blocked. Delivery optics 22,24,26 which may comprise light guiding elements, such as mirrors, divergence or emittance converting elements or the like, schematically represented here in Figure 1 by simple flat mirrors and curved mirrors, guide the laser pulses 16,18 to a reaction or interaction volume. Each laser pulse 16,18 has its own light path towards the target device 28. The delivery optics comprises a pair of movable mirrors forming a delay line 24 for the second laser pulse 18. When the delay line 24 is moved in direction of the double arrow, the length of the light path of second laser pulse 18 is changed and, hence, its arrival time at the target device 28 can be influenced. Please note that the two mirrors 22 can also be a delay line if they are accordingly movable. Each of the laser pulses is focused with the aid of a focussing mirror 26, preferably an f/18 off-axis parabolic mirror or spherical mirror, in the interaction zone of the target device 28, onto the target device 28 along their respective light paths. the laser pulses 16,18 arrive anti-collinear or in head-on at the target device 28 in this preferred embodiment. The first and the second laser pulse 16,18 overlap, collide or interfere in the interaction zone. In the interaction zone, the electron source, there is a fluid jet, in preferred embodiment a supersonic Helium gas jet with 3 mm diameter. The high-energy electrons 30 are produced in the interaction zone of the target device 28 preferably positioned in the focus or close to the focus, for instance in the Rayleigh range of the focus, of the laser pulses 16,18. Upon irradiation an initial electron density in the plasma of 3*10^18 electrons / cm^3 is created. The high-energy electrons 30 are propagating along the light paths of the colliding laser pulses.

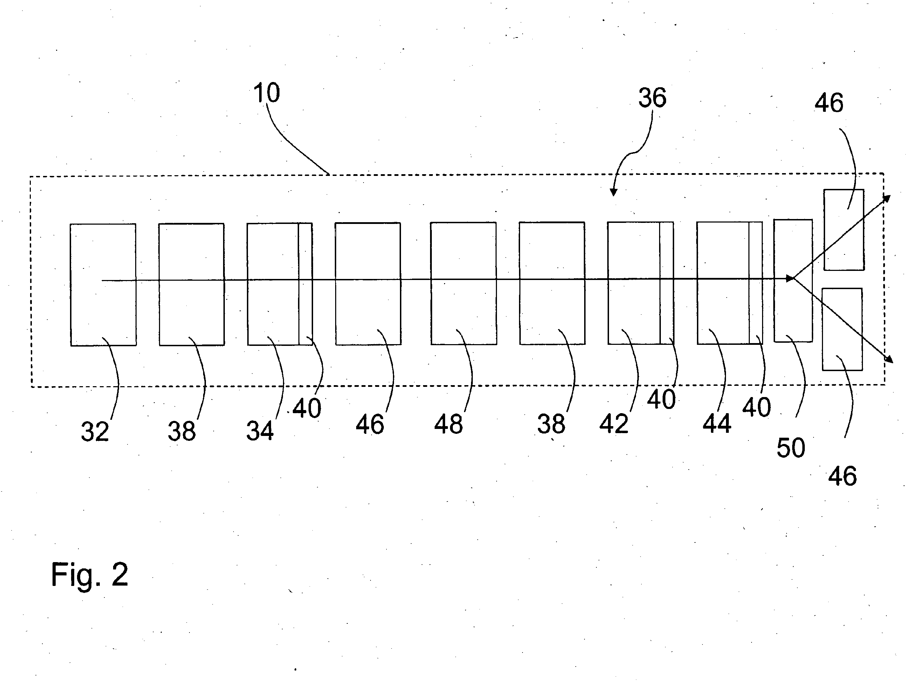

In Figure 2 a scheme of the preferred embodiment of the laser system 10 used in the device and method according to the invention is shown. The laser system 10 is a so-called double-CPA laser system. It operates in chirped-pulse amplification mode at 820 nm (nanometer) central wavelength. A mode-coupled oscillator 32 comprises a Titanium:Sapphire crystal which is pumped by an Argon-ion laser. The oscillator 32 output consists of femtosecond pulses, in particular essentially 15 fs long, with an energy of 2 nJ with a repetition rate of approximately 88 MHz. The oscillator 32 pulses are stretched by a pair of optical gratings in stretcher 38 (pulse chirping) and an acousto-optical modulator is used afterwards to select individual pulses at a frequency of 10 Hz out of the high-frequency pulse train leaving the oscillator 32 and the stretcher 38. After that pulses essentially 400 ps long and with an energy of about 500 pJ enter an 8-pass pre-amplifier 34. The pre-amplifier 34 is pumped by a frequency-doubled pulsed Nd:YAG laser with 200mJ energy per pulse at a frequency of 10 Hz. Stretcher 38 and pre-amplifier 34 are optically isolated using an arrangement of a Pockels cell between polarizers. The output of pre-amplifier 34 passes through a spatial filter 40 (afocal x4) and conveys an energy of 2mJ per pulse. Now the 10 Hz pulse train is partially or totally recompressed (compressor 46, pulse dechirping) and passes a device 48 for shaping the temporal intensity profile (preferred topology after the pre-amplification stage). It is advantageous to increase the laser contrast, meaning the difference in intensity between the maximum and the leading edge (of the wing) of the pulse, by shaping the temporal intensity profile right after the pre-amplification stage. The device 48 is followed by a second stretcher 38 (pulse chirping) and by a main amplifier 36. The main amplifier 36 comprises a 5-pass first power amplifier 42 pumped by a frequency-doubled pulsed Nd:YAG laser with 1J energy per pulse at 10 Hz. The pulses amplified to 200 mJ energy pass through a spatial filter 40, preferably a vacuum spatial filter (afocal x4) and enter a 4-pass second power amplifier 44 of the main amplifier 36. The crystal of the second power amplifier 44 is contained in a cryogenic chamber at 120 K temperature. Several frequency-doubled pulsed Nd:YAG lasers pump this amplification stage: Three lasers at 1.7 J, three lasers at 1.5 J, an one laser at 1.7 J are used. This arrangement results in an output of pulses being 400 ps long and having an energy of 2.5 J. After the second amplification a spatial filter 40, preferably a vacuum spatial filter (afocal x1) is traversed. The amplified laser pulse enters a tuneable beam splitter 50, e.g. a beam splitter whose splitting ratio can be adjusted or changed, creating a first and a second laser pulse. These pulses are eventually each compressed in a vacuum compressor 46 (pulse dechirping) using a pair of optical gratings reaching pulses being 40 fs to 25 fs long (full width half maximum) and having a maximal energy of the order of 1.5 J. The waist'of the focal spot is 18 µm, resulting in vacuum focused intensities of the order of 2 x 10^18 W/cm^2, which corresponds to a normalised laser vector potential of 1.4, reaching on-target energies of 1.3 J.

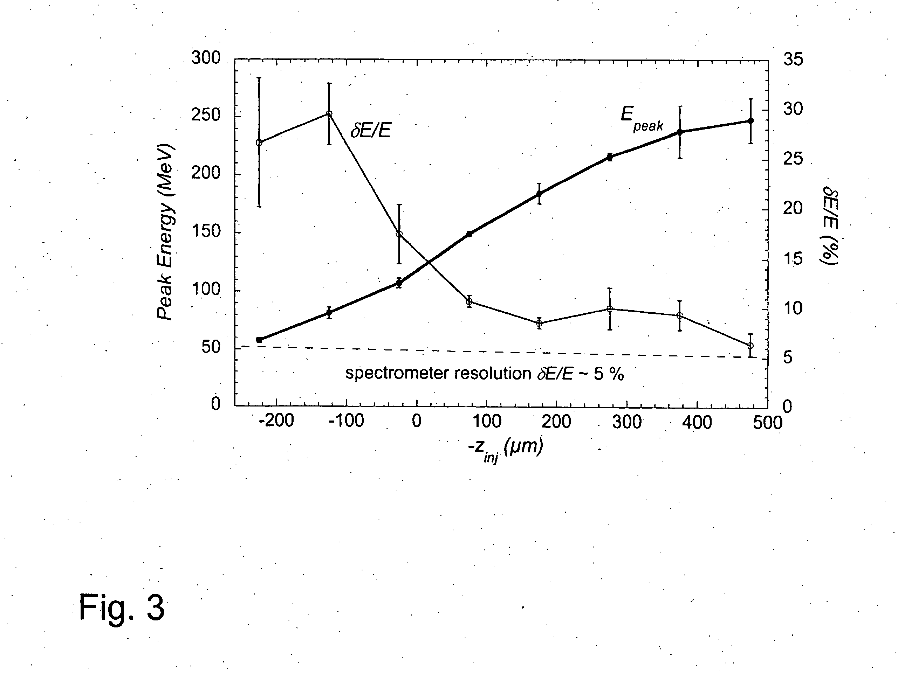

In Figure 3 measured results achieved using a setup having the topology shown in Figure 1 are displayed. The graph shows the peak energy of the electrons in MeV (mega electronvolts) as a function of the delay length in µm (micrometers) between the two overlapping laser pulses (left scale on the abscissa). The graph shows also the energy spread or the energy distribution width in relation to the peak energy (relative energy spread δE/E) in % as a function of the delay length in µm (micrometers) (right abscissa). The spectrometer resolution used for the measurements in this embodiment is about 5%. With increasing overlap the peak energy is rising monotonously and steeply reaching remarkable 250 MeV. Parallel to that the relative energy spread is dropping monotonously and sharply reaching the limit of the spectrometer resolution. By changing the delay and in consequence the degree of the overlap of the two colliding pulses in the interaction zone, the peak energy of the generated high-energy electrons is tuneable. A quasi-monoenergetic electron bunch, pulse or beam can be created.

### REFERENCE NUMERAL LIST

- 10: laser system
- 12: amplified laser pulse
- 14: beamsplitter
- 16: first laser pulse
- 18: second laser pulse
- 20: blocking device
- 22: delivery optics
- 24: delay line
- 26: focussing mirror
- 28: target device
- 30: high-energy electrons
- 32: oscillator
- 34: pre-amplifier
- 36: main amplifier
- 38: stretcher
- 40: spatial filter
- 42: first power amplifier
- 44: second power amplifier
- 46: compressor
- 48: device for shaping the temporal intensity profile
- 50: tuneable beam splitter

## Claims

1. A device for creating high-energy electrons (30), comprising:
- a laser system (10) producing laser pulses with a pulse length shorter than 1000 fs and capable to be focused to peak intensities greater than 10^17 W/cm^2,
- at least one target device (28) with an interaction zone arranged to be exposed to laser pulses,
- delivery optics (22,24,26) having components being capable to guide a first laser pulse (16) and a second laser pulse (18) which both are produced by the laser system (10) to the at least one target device (28) in such a way that the first laser pulse (16) and the second laser pulse (18) at least partially overlap in the interaction zone of the target device thereby releasing high-energy electrons (30) from the interaction zone, **characterised in that**
the location of the target device (28) being tuneable for changing the location of the interaction zone where the first laser pulse (16) and the second laser pulse (18) at least partially overlap.

2. A device for creating high-energy electrons (30) according to claim 1,
**characterised in that**
the delivery optics (22,24,26) being tuneable for changing the temporal and/or spatial overlap of the first laser pulse (16) and the second laser pulse (18) in the interaction zone.

3. A device for creating high-energy electrons (30) according to claim 1,
**characterised in that**
the first laser pulse (16) and the second laser pulse (18) are substantially counter-propagating through the interaction zone.

4. A device for creating high-energy electrons (30) according to claim 1,
**characterised by**
further components of the delivery optics capable to guide at least a third laser pulse which is also produced by the laser system (10) to the at least one target device (28) in such a way that the third laser pulse either arrives before the first laser pulse (16) and the second laser pulse (18) or at least partially overlaps in the interaction zone of the target device with the first laser pulse (16) and/or the second laser pulse (18).

5. A device for creating high-energy electrons (30) according to claim 1,
**characterised by**
a blocking device (20) for avoiding feedback of laser energy and/or entry of laser energy counter to a light path of a laser pulse through the delivery optics (22,24,26) into the laser system (10) or into parts of the laser system (10) upstream from a compressor (46).

6. A device for creating high-energy electrons (30) according to claim 1,
**characterised in that**
the laser system (10) is a chirped pulse amplification facility of a self mode-locked Ti:Sapphire laser with output energy greater than 0.6 J, output power greater than 20 TW capable of emitting laser pulses shorter than 40 fs.

7. A device for creating high-energy electrons (30) according to claim 1,
**characterised in that**
the delivery optics (22,24,26) comprises a delay line (24) for adjusting the arrival time of one of the first laser pulse (16) and the second laser pulse (18) with respect to the other of the first and the second laser pulse (16,18) in the interaction zone.

8. A device for creating high-energy electrons (30) according to claim 1,
**characterised by**
a control unit capable to change the temporal and/or spatial overlap of the first and the second laser pulse in the interaction zone by changing the length and/or the run of at least one of the light paths of the first and the second laser pulses (16,18) through the delivery optics (22,24,26).

9. A device for creating high-energy electrons (30) according to claim 1,
**characterised in that**
the first laser pulse (16) and the second laser pulse (18) are created out of one amplified laser pulse (12) of the laser system (10) by splitting the amplified laser pulse (12) into two pulses before pulse compression.

10. A device for creating high-energy electrons (30) according to claim 1,
**characterised in that**
the laser system (10) comprises a tuneable beam splitter (50) acting on the amplified laser pulse before the compressors (14,46).

11. A device for creating high-energy electrons (30) according to claim 1,
**characterised in that**
the peak intensity of the first laser pulse (16) is more than 3 times, or greater than the peak intensity of the second laser pulse (18) and/or the normalised vector potential of the first laser pulse (16) is more than 1.5 times, or greater than the normalised vector potential of the second laser pulse (18).

12. A device for creating high-energy electrons (30) according to claim 1,
**characterised in that** the target device (28) in the interaction zone comprises a gas target and/or a plasma target, in particular comprising hydrogen or helium, with adjustable density for which no self injection by one laser pulse (16,18) is dominant.

13. A method for creating high-energy electrons (30), comprising:
- Producing laser pulses with a pulse length shorter than 1000 fs and capable to be focused to peak intensities greater than 10^17 W/cm^2,
- Providing at least one target device (28) with an interaction zone arranged to be exposed to laser pulses,
- Guiding a first laser pulse (16) and a second laser pulse (18) which both are produced by the laser system (10) to the at least one target device (28) in such a way that the first laser pulse (16) and the second laser pulse (18) at least partially overlap in an interaction zone of the target device (28) thereby releasing high-energy electrons (30) from the interaction zone,
**characterized by**
Tuning the location of the target device (28) for changing the location of the interaction zone where the first laser pulse (16) and the second laser pulse (18) at least partially overlap.

## Patentansprüche

1. Vorrichtung zur Erzeugung hochenergetischer Elektronen (30), umfassend:
- ein Lasersystem (10), das Laserpulse mit einer Pulslänge kürzer als 1000 fs erzeugt, die sich auf Spitzenintensitäten größer als 10¹⁷ W/cm² fokussieren lassen,
- wenigstens eine Target-Vorrichtung (28) mit einer Wechselwirkungszone, die dafür ausgelegt ist, Laserpulsen ausgesetzt zu werden,
- eine Strahlführungsoptik (22, 24, 26) mit Komponenten, die fähig sind, einen ersten Laserpuls (16) und einen zweiten Laserpuls (18), die beide vom Lasersystem (10) erzeugt sind, zu der wenigstens einen Target-Vorrichtung (28) zu führen, derart, dass der erste Laserpuls (16) und der zweite Laserpuls (18) in der Wechselwirkungszone der Target-Vorrichtung wenigstens teilweise überlappen, wodurch hochenergetische Elektronen (30) aus der Wechselwirkungszone freigesetzt werden,
**dadurch gekennzeichnet, dass**
der Ort der Target-Vorrichtung (28) einstellbar ist, um den Ort der Wechselwirkungszone, wo der erste Laserpuls (16) und der zweite Laserpuls (18) wenigstens teilweise überlappen, zu ändern.

2. Vorrichtung zur Erzeugung hochenergetischer Elektronen (30) nach Anspruch 1,
**dadurch gekennzeichnet, dass**
die Strahlführungsoptik (22, 24, 26) einstellbar ist, um die zeitliche und/oder räumliche Überlappung des ersten Laserpulses (16) und des zweiten Laserpulses (18) in der Wechselwirkungszone zu ändern.

3. Vorrichtung zur Erzeugung hochenergetischer Elektronen (30) nach Anspruch 1,
**dadurch gekennzeichnet, dass**
der erste Laserpuls (16) und der zweite Laserpuls (18) sich durch die Wechselwirkungszone im Wesentlichen gegenläufig ausbreiten.

4. Vorrichtung zur Erzeugung hochenergetischer Elektronen (30) nach Anspruch 1,
**gekennzeichnet durch**
weitere Komponenten der Strahlführungsoptik, die fähig sind, wenigstens einen dritten Laserpuls, der ebenfalls vom Lasersystem (10) erzeugt ist, zu der wenigstens einen Target-Vorrichtung (28) zu führen, derart, dass der dritte Laserpuls entweder vor dem ersten Laserpuls (16) und dem zweiten Laserpuls (18) ankommt oder in der Wechselwirkungszone der Target-Vorrichtung wenigstens teilweise mit dem ersten Laserpuls (16) und/oder dem zweiten Laserpuls (18) überlappt.

5. Vorrichtung zur Erzeugung hochenergetischer Elektronen (30) nach Anspruch 1,
**gekennzeichnet durch**
eine Sperrvorrichtung (20) zur Vermeidung einer Rückkopplung von Laserenergie und/oder eines Eintritts von Laserenergie entgegen einem Lichtweg eines Laserpulses **durch** die Strahlführungsoptik (22, 24, 26) in das Lasersystem (10) oder in Teile des Lasersystems (10), die einer Kompressionsvorrichtung (46) vorgelagert sind.

6. Vorrichtung zur Erzeugung hochenergetischer Elektronen (30) nach Anspruch 1,
**dadurch gekennzeichnet, dass**
das Lasersystem (10) ein sogenanntes Chirped Pulse Amplification-System eines selbstmodengekoppelten Ti:Saphir-Lasers mit einer Ausgangsenergie von mehr als 0,6 J, einer Ausgangsleistung von mehr als 20 TW ist, das fähig ist, Laserpulse auszusenden, die kürzer als 40 fs sind.

7. Vorrichtung zur Erzeugung hochenergetischer Elektronen (30) nach Anspruch 1,
**dadurch gekennzeichnet, dass**
die Strahlführungsoptik (22, 24, 26) ein Verzögerungselement (24) zum Einstellen der Ankunftszeit eines des ersten Laserpulses (16) und des zweiten Laserpulses (18), in Bezug auf den anderen des ersten und zweiten Laserpulses (16, 18), in der Wechselwirkungszone umfasst.

8. Vorrichtung zur Erzeugung hochenergetischer Elektronen (30) nach Anspruch 1,
**gekennzeichnet durch**
eine Steuereinheit, die fähig ist, die zeitliche und/oder räumliche Überlappung des ersten und des zweiten Laserpulses in der Wechselwirkungszone durch Ändern der Länge und/oder des Verlaufs wenigstens eines der Lichtwege des ersten und des zweiten Laserpulses (16, 18) **durch** die Strahlführungsoptik (22, 24, 26) zu ändern.

9. Vorrichtung zur Erzeugung hochenergetischer Elektronen (30) nach Anspruch 1,
**dadurch gekennzeichnet, dass**
der erste Laserpuls (16) und der zweite Laserpuls (18) aus einem verstärkten Laserpuls (12) des Lasersystems (10) erzeugt werden und zwar durch Teilen des verstärkten Laserpulses (12) in zwei Pulse, bevor eine Pulskompression erfolgt.

10. Vorrichtung zur Erzeugung hochenergetischer Elektronen (30) nach Anspruch 1,
**dadurch gekennzeichnet, dass**
das Lasersystem (10) einen einstellbaren Strahlteiler (50) umfasst, der vor den Kompressionsvorrichtungen (14, 46) auf den verstärkten Laserpuls einwirkt.

11. Vorrichtung zur Erzeugung hochenergetischer Elektronen (30) nach Anspruch 1,
**dadurch gekennzeichnet, dass**
die Spitzenintensität des ersten Laserpulses (16) mehr als 3-mal oder größer als die Spitzenintensität des zweiten Laserpulses (18) ist und/oder das normierte Vektorpotential des ersten Laserpulses (16) mehr als 1,5-mal oder größer als das normierte Vektorpotential des zweiten Laserimpulses (18) ist.

12. Vorrichtung zur Erzeugung hochenergetischer Elektronen (30) nach Anspruch 1,
**dadurch gekennzeichnet, dass** die Target-Vorrichtung (28) in der Wechselwirkungszone ein Gas-Target und/oder ein Plasma-Target, insbesondere Wasserstoff oder Helium enthaltend, mit einer einstellbaren Dichte umfasst, bei der keine Selbstinjektion durch einen Laserpuls (16, 18) vorherrschend ist.

13. Verfahren zur Erzeugung hochenergetischer Elektronen (30), umfassend:
- Erzeugen von Laserpulsen mit einer Pulslänge kürzer als 1000 fs, die sich auf Spitzenintensitäten größer als 10¹¹ W/cm² fokussieren lassen,
- Bereitstellen wenigstens einer Target-Vorrichtung (28) mit einer Wechselwirkungszone, die dafür ausgelegt ist, Laserpulsen ausgesetzt zu werden,
- Führen eines ersten Laserpulses (16) und eines zweiten Laserpulses (18), die beide vom Lasersystem (10) erzeugt sind, zu der wenigstens einen Target-Vorrichtung (28), derart, dass der erste Laserpuls (16) und der zweite Laserpuls (18) in einer Wechselwirkungszone der Target-Vorrichtung (28) wenigstens teilweise überlappen, wodurch hochenergetische Elektronen (30) aus der Wechselwirkungszone freigesetzt werden,
**gekennzeichnet durch**
Einstellen des Orts der Target-Vorrichtung (28), um den Ort der Wechselwirkungszone, wo der erste Laserpuls (16) und der zweite Laserpuls (18) wenigstens teilweise überlappen, zu ändern.

## Revendications

1. Dispositif pour générer des électrons de haute énergie (30), comprenant :
- un système laser (10) générant des impulsions laser à des longueurs d'impulsion inférieures à 1000 fs et pouvant être focalisées à des intensités pics de plus de 10¹⁷ W/cm²,
- au moins un dispositif cible (28) comprenant une zone d'interaction conçue pour être exposée aux impulsions laser,
- une optique d'acheminement (22, 24, 26) possédant des composants capables de guider une première impulsion laser (16) et une seconde impulsion laser (18) toutes deux produites par le système laser (10) vers ledit au moins un dispositif cible (28) de sorte que la première impulsion laser (16) et la seconde impulsion laser (18) se chevauchent au moins partiellement dans la zone d'interaction du dispositif cible, libérant ainsi des électrons de haute énergie (30) de la zone d'interaction,
**caractérisé en ce que** :
l'emplacement du dispositif cible (28) est ajustable afin de modifier la zone d'interaction où la première impulsion laser (16) et la seconde impulsion laser (18) se chevauchent au moins partiellement.

2. Dispositif pour générer des électrons de haute énergie (30) selon la revendication 1,
**caractérisé en ce que**
l'optique d'acheminement (22, 24, 26) est ajustable afin de changer le chevauchement temporel et/ou spatial de la première impulsion laser (16) et de la seconde impulsion laser (18) dans la zone d'interaction.

3. Dispositif pour générer des électrons de haute énergie (30) selon la revendication 1,
**caractérisé en ce que**
la première impulsion laser (16) et la seconde impulsion laser (18) se propagent essentiellement en sens inverse dans la zone d'interaction.

4. Dispositif pour générer des électrons de haute énergie (30) selon la revendication 1,
**caractérisé par**
d'autres composants de l'optique d'acheminement capables de guider au moins une troisième impulsion laser également produite par le système laser (10) vers ledit au moins un dispositif cible (28) de sorte que la troisième impulsion laser arrive avant la première impulsion laser (16) et la seconde impulsion laser (18) ou chevauche au moins partiellement, dans la zone d'interaction du dispositif cible, la première impulsion laser (16) et/ou la seconde impulsion laser (18).

5. Dispositif pour générer des électrons de haute énergie (30) selon la revendication 1,
**caractérisé par**
un dispositif de blocage (20) afin d'éviter un retour d'énergie laser et/ou une entrée d'énergie laser à l'encontre d'un trajet de lumière d'une impulsion laser via l'optique d'cheminement (22, 24, 26) dans le système laser (10) ou dans des parties du système laser (10) en amont d'un compresseur (46).

6. Dispositif pour générer des électrons de haute énergie (30) selon la revendication 1,
**caractérisé en ce que**
le système laser (10) est un équipement d'amplification d'impulsion modulée d'un laser Ti:saphir verrouillé en mode automatique dont l'énergie de sortie est supérieure à 0,6 J, la puissance de sortie est supérieure à 20 TW, et pouvant émettre des impulsions laser inférieures à 40 fs.

7. Dispositif pour générer des électrons de haute énergie (30) selon la revendication 1,
**caractérisé en ce que**
l'optique d'acheminement (22, 24, 26) comprend une ligne de retard (24) afin d'ajuster le temps d'arrivée de l'une de la première impulsion laser (16) et de la seconde impulsion laser (18) par rapport à l'autre de la première impulsion laser (16) et de la seconde impulsion laser (18) dans la zone d'interaction.

8. Dispositif pour générer des électrons de haute énergie (30) selon la revendication 1,
**caractérisé par**
une unité de commande capable de changer le chevauchement temporel et/ou spatial de la première et de la seconde impulsion laser dans la zone d'interaction en modifiant la longueur et/ou la course d'au moins un des trajets de lumière des première et seconde impulsions laser (16, 18) à travers l'optique d' acheminement (22, 24, 26).

9. Dispositif pour générer des électrons de haute énergie (30) selon la revendication 1,
**caractérisé en ce que**
la première impulsion laser (16) et la seconde impulsion laser (18) sont générées à partir d'une impulsion laser amplifiée (12) du système laser (10) en divisant l'impulsion laser amplifiée (12) en deux impulsions avant la compression d'impulsions.

10. Dispositif pour générer des électrons de haute énergie (30) selon la revendication 1,
**caractérisé en ce que**
le système laser (10) comprend un diviseur de faisceau ajustable (50) agissant sur l'impulsion laser amplifiée avant les compresseurs (14, 46).

11. Dispositif pour générer des électrons de haute énergie (30) selon la revendication 1,
**caractérisé en ce que**
l'intensité pic de la première impulsion laser (16) représente plus de 3 fois ou plus de l'intensité pic de la seconde impulsion laser (18) et/ou le potentiel de vecteur normalisé de la première impulsion laser (16) représente plus de 1,5 fois ou plus du potentiel de vecteur normalisé de la seconde impulsion laser (18).

12. Dispositif pour générer des électrons de haute énergie (30) selon la revendication 1,
**caractérisé en ce que**
le dispositif cible (28) dans la zone d'interaction comprend une cible à gaz et/ou une cible plasma comprenant notamment de l'hydrogène ou de l'hélium, avec une densité ajustable pour laquelle aucune injection automatique par une impulsion laser (16, 18) n'est dominante.

13. Procédé pour générer des électrons de haute énergie (30), consistant à :
- générer des impulsions laser à des longueurs d'impulsion inférieures à 1000 fs et pouvant être focalisées à des intensités pics de plus de 10¹⁷ W/cm²,
- utiliser au moins un dispositif cible (28) comprenant une zone d'interaction conçue pour être exposée aux impulsions laser,
- guider une première impulsion laser (16) et une seconde impulsion laser (18) toutes deux produites par le système laser (10) vers ledit au moins un dispositif cible (28) de sorte que la première impulsion laser (16) et la seconde impulsion laser (18) se chevauchent au moins partiellement dans une zone d'interaction du dispositif cible (28), libérant ainsi des électrons de haute énergie (30) de la zone d'interaction,
**caractérisé par**
l'ajustement de l'emplacement du dispositif cible (28) afin de modifier l'emplacement de la zone d'interaction où la première impulsion laser (16) et la seconde impulsion laser (18) se chevauchent au moins partiellement.
